# EUROPEAN PATENT SPECIFICATION

(11) **EP 2 988 616 B1**
(45) Date of publication and mention of the grant of the patent: **17.10.2018**
(21) Application number: 14712908.4
(22) Date of filing: 05.03.2014
(51) Int. Cl.: A23L 33/10, A61K 35/74, A61P 1/08

(54) **TREATMENT OF CHRONIC IDIOPATHIC EMESIS IN FELINES AND CANINES**
BEHANDLUNG VON CHRONISCHER IDIOPATHISCHER EMESIS BEI KATZEN UND HUNDEN
TRAITEMENT DE VOMISSEMENT IDIOPATHIQUE CHRONIQUE CHEZ DES FÉLINS ET DES CHIENS

(30) Priority: 15.03.2013 US 201361792657 P
(43) Date of publication of application: 02.03.2016
(73) Proprietor: Mars, Incorporated, McLean, VA 22101 (US)
(72) Inventor: PARK, Jean, Soon, Cincinnati, Ohio 45202 (US)
(74) Representative: Nony
(86) International application number: PCT/US2014/020522
(87) International publication number: WO 2014/149716

(56) References cited:
- CN-A- 102 440 375
- US-A1- 2006 008 511
- O'MAHONY D ET AL: "Portrait of a canine probiotic Bifidobacterium-From gut to gut", VETERINARY MICROBIOLOGY, ELSEVIER BV, NL, vol. 139, no. 1-2, 20 October 2009 (2009-10-20), pages 106-112, XP026642093, ISSN: 0378-1135, DOI: 10.1016/J.VETMIC.2009.05.002 [retrieved on 2009-05-19]
- R L Kelley ET AL: "Clinical Benefits of Probiotic Canine-Derived Bifidobacterium animalis Strain AHC7 in Dogs with Acute Idiopathic Diarrhea* C L I NI CA L RE LE V A NCE", , 21 October 2009 (2009-10-21), XP055116332, Retrieved from the Internet: URL:https://s3.amazonaws.com/assets.prod.v etlearn.com/mmah/10/23a28945ae47e5b3fb9b02 2810e139/fileVTX_10_03_121.pdf [retrieved on 2014-05-05]

## Description

### FIELD OF THE INVENTION

The present invention is to the field of probiotics and their use for treatment of chronic idiopathic emesis in canines and felines.

### BACKGROUND OF THE INVENTION

Emesis is an animal's defense mechanism against ingested toxins or foreign materials. Emesis is a process that includes nausea, retching and vomiting and is a common symptom of many diseases such as food allergies, food poisoning, irritable bowel syndrome, Crohn's disease, colitis, cancer, gastritis, and intestinal ulcers. Also, it can be a side effect of medical treatments such as chemotherapy, radiation, and anesthesia. In felines, including domestic cats, emesis can also be due to hairballs. In most cases the incidence of emesis can be explained by a veterinarian based on obvious signs of disease, and so the emesis can be easily treated.

However, in a number of cases, canines and felines suffer from frequent emesis in the absence of any signs of disease or cause. This is referred to as chronic idiopathic emesis. Incidents of chronic idiopathic emesis can be frequent and seemingly random and cause distress to the canine/feline and owner. Symptoms of chronic idiopathic emesis can include frequent and unexplainable vomiting of food. When emesis is prolonged or severe, and goes untreated, it may lead to complications such as dehydration and malnutrition as well as chemical imbalances caused by loss of body fluid and nutrients. The added stress associated with these health conditions can result in more rapid aging and the development of age-related chronic diseases. Due to the complex nature of the emesis mechanism and the lack of knowledge in the causes of vomiting (including vomiting of food), current veterinary/medical practice is usually aimed at eliminating the symptoms of emesis rather than controlling the condition.

There is a need in the art for means to reduce the incidents of chronic idiopathic emesis in felines and canines, especially domestic cats and dogs. There is also a need in the art for means to reduce incidents of chronic idiopathic emesis in felines and canines that can be easily and efficiently administered to the animal.

The Inventors have surprisingly found that chronic idiopathic emesis in felines and canines can be treated using probiotics, especially probiotic *bifidobacterium spp.*

The use of probiotics for the reduction of incidents of vomiting in cats has previously been disclosed in US2006/0008511A1. However, US2006/0008511A1 addresses the problem of reducing diarrhea, vomiting, body odor and flatulence in felines, caused by known gastrointestinal diseases, such as imbalance of gut flora. US2006/0008511A1 does not address the separate problem of reducing chronic idiopathic emesis in felines, which as discussed earlier is not the result of gastrointestinal or other disease in the feline.

The article entitled "Clinical Benefits of Probiotic Canine-Derived *Bifidobacterium animalis* Strain AHC7 in Dogs with Acute Idiopathic Diarrhea" by R.L. Kelley *et. al.* discloses that *Bifidobacterium animals* AHC7 is effective in treating acute idiopathic diarrhea in dogs, but does not address the problem of idiopathic emesis.

CN 102440375 A discloses a composition comprising *Bifidobacterium infantis* in being effective against diarrhea, vomiting, constipation, anorexia and dyspepsia in children, but does not disclose canines and felines nor treatment of idiopathic emesis.

The article entitled "Portrait of a canine probiotic Bifidobacterium from gut to gut" by O'Mahony D *et. al*._discloses *Bifidobacterium animalis* AHC7 having potential to improve canine gastrointestinal health.

The Inventors surprisingly found that felines and canines that suffered from chronic idiopathic emesis showed reduced incidents of emesis following treatment with probiotics as compared to felines and canines suffering from chronic idiopathic emesis that were fed a control diet without probiotics. The Inventors surprisingly found that the use of the probiotic according to the present invention provided a simple and efficient means for administrating a treatment for chronic idiopathic emesis to felines and canines.

In humans, repeated bouts of unexplained emesis may be associated with cyclic vomiting syndrome. Some human infants are also prone to emesis which does not appear to correlate to overeating or other common causes, although the condition in infants does not always progress to cyclic vomiting syndrome in childhood or beyond. Probiotics may also be helpful in reducing incidences of emesis in humans prone to emesis, such as humans with a history of cyclic vomiting syndrome, or human infants with a history of unexplained emesis.

### SUMMARY OF THE INVENTION

The present invention is as claimed in the claims.

The present invention concerns a probiotic for use in the prevention, alleviation, or treatment of unexplained emesis, and a method of preventing, alleviating, or treating unexplained emesis in mammals. In some aspects, the present invention concerns the a probiotic for the use in treatment of chronic idiopathic emesis in canines and felines. The probiotic for use according to the invention comprises *Bifidobacterium spp.,* the bifidobacterium is *Bifidobacterium animalis AHC7.*

### DETAILED DESCRIPTION OF THE INVENTION

### Definitions

As used herein, the articles including "the", "a", and "an", when used in a claim or in the specification, are understood to mean one or more of what is claimed or described.

As used herein, the terms "include", "includes", and "including" are meant to be nonlimiting.

As used herein, the term "plurality" means more than one.

As used herein, the term "animal" means a feline or canine unless otherwise stated and the term "pet" means a domestic dog (canines) or cat (felines) unless otherwise stated. As used herein, the terms "animal feed", "animal feed compositions", "animal feed kibble", "pet food", or "pet food composition" all mean a composition intended for ingestion by a pet. Pet foods can include, without limitation, nutritionally balanced compositions suitable for daily feed, as well as supplements and/or treats, which may or may not be nutritionally balanced. As used herein, the term "nutritionally balanced" means that a composition, such as pet food, has known required nutrients to sustain life in proper amounts and proportions based on recommendations of recognized authorities, including governmental agencies, such as, but not limited to, Unites States Food and Drug Administration's Center for Veterinarian Medicine, the American Feed Control Officials Incorporated, in the field of pet nutrition, except for the additional need for water.

### Use of the Probiotic

The present invention is to a probiotic for use in the treatment of chronic idiopathic emesis in canines and felines, wherein the probiotic comprises *Bifidobacterium spp* and wherein the bifidobacterium is *Bifidobacterium animalis AHC7.*

Chronic idiopathic emesis is defined as unexplainable emesis or vomiting that occurs in the absence of any clinical indicators of illness or distress in the animal. Chronic idiopathic emesis can be random and frequent and can include frequent and unexplainable vomiting of food. Without wishing to be bound by theory, chronic idiopathic emesis is not the same as the occurrence of emesis or vomiting due to clear indicators of illness or distress, for example, but not limited to, bacterial or viral infection, hormonal imbalance, or ingestion of poison. Whilst the prior art has addressed the reduction of vomiting explainable by clear clinical indicators, it has not addressed the reduction of chronic idiopathic emesis.

In one embodiment, the present invention is to *Bifidobacterium animalis* AHC7 for use in the treatment of chronic idiopathic emesis in felines. The present invention may be to Bifidobacterium animalis AHC7 for use in the treatment of chronic idiopathic emesis in domestic cats.

In one embodiment, the present invention is to Bifidobacterium animalis AHC7 for use in the treatment of chronic idiopathic emesis in canines. The present invention may be to Bifidobacterium animalis AHC7 for use in the treatment of chronic idiopathic emesis in domestic dogs.

In one embodiment, the present invention is to Bifidobacterium animalis AHC7 for use in the treatment of chronic idiopathic emesis in domestic cats, domestic dogs or both.

The probiotic for use according to the invention comprises *Bifidobacterium spp.,* the bifidobacterium is *Bifidobacterium animalis AHC7* and is described in more detail below.

The probiotic of the invention may be administered to the canine or feline at least once per day, or even at least twice per day, or even at least three times per day, or even more than three times per day for use in the treatment of chronic idiopathic emesis.

The probiotic may be administered to a canine or feline at a concentration of between 1 × 106 and 1 × 1012 CFU/feline 5 or canine/day, or even between 1 × 108 and 1 × 1010 CFU/feline or canine/day. Colony forming units (CPU) is determined using the method provided as part of the European Pharmacopoeial Methods, 2003, Section 2.6.12.

The probiotic may be administered in the form of the probiotic itself, a medicament comprising the probiotic, a food composition comprising the probiotic or a mixture thereof. If administered as a medicament, then the feline or canine may be administered the medicament at least once per day. If administered in a food composition, the feline or canine can be fed at regular times at least once per day. Alternatively, the feline or canine may be fed *ad libitum,* i.e. a large portion of food is left for the feline or canine at the start of the day and the feline or canine eat quantities of the food during the day as wanted.

### Probiotic

A probiotic is a dietary supplement that contains live microorganisms, especially live bacteria or fungi.

The probiotic comprises *Bifidobacterium spp,* wherein the bifidobacterium is *Bifidobacterium animalis* AHC 7. *B. animalis* AHC7 is a well known strain that has been described in the scientific literature (O'Mahony et al., BMC Immunology, 2010, 11:63) and is readily available to those skilled in the art.

The probiotic may be comprised in a food composition or be in the form of a medicament to be ingested by the animal. A medicament may be ingested separately to regular food ingested by the animal or may be added to a food preparation for the animal.

In one embodiment, the probiotic for use according to the present invention can have a viable probiotic microorganism count of at least 1 × 103 colony forming units (CFU) per gram of probiotic, or at
least about 1 × 104 CFU per gram of probiotic, or at least about 1 × 105 CFU per gram of probiotic or at least about 1 × 106 CFU per gram of probiotic. For example, the composition may have a viable probiotic microorganism count of up to about 1 × 1015 CFU per gram of probiotic, up to about 1 × 1014 CFU per gram of probiotic, or up to about 1 × 1013 CFU per gram of probiotic, or up to about 1 × 1012 CFU per gram of probiotic. CFU is determined using the method provided as part of the European Pharmacopoeial Methods, 2003, Section 2.6.12.

The probiotic for use according to the present invention can be prepared using common techniques known in the art.

### Medicament

The probiotic maybe in the form of a medicament. In this 5 embodiment, the medicament may be in dry form, for example a tablet, or powder (comprised in a sachet). The medicament may be in the form of a capsule comprising a probiotic in dry form.

The medicament may be administered separately to regular food ingested by the animal or may be added to a food preparation for the animal.

The medicament may comprise other components provide other desirable benefits such as stability or taste.

The medicament may comprise at least about 0.001%, alternatively at least about 0.01%, alternatively at least about 0,1%, alternatively at least about 0.5%, and alternatively at least about 1% of the probiotic, by weight of the medicament. As further examples, the medicament may comprise about 99% or less, alternatively about 75% or less, alternatively about 50% or less, alternatively about 25% or less, alternatively about 10% or less, and alternatively about 5% or less of the probiotic, by weight of the medicament.

The medicament can be prepared using common techniques known in the art.

### Food compositions

Discloses is the use of probiotics in a food composition that is intended for ingestion and that comprises the probiotic. The food composition may be intended for a feline or canine, another companion animal, or a human. Compositions include foods intended to supply necessary dietary requirements, as well as treats (e.g., biscuits) or other food supplements. Optionally, the composition herein may be a dry composition (for example, kibble). Alternatively or additionally, the food composition is a supplement, such as a gravy, yogurt, powder, suspension, chew, treat (e.g. biscuits) or any other delivery form. The food composition may be in the form of kibble, biscuit, gravy, yogurt, powder, suspension, chew or mixture thereof.

Moreover, in one embodiment the composition can be nutritionally balanced, such as a pet food kibble. In another embodiment, the composition is not nutritionally balanced, such as a supplement, treat, or other delivery form for a pet. Nutritionally balanced pet foods and supplements, and the manufacturing processes thereof, are well known in the art.

In one embodiment, the probiotic for use according to the present invention can have a viable probiotic microorganism count of at least about 1x103 CFU per gram of food composition, or at least about 1x104 CFU per gram of probiotic, or at least about 1x105 CFU per gram of food composition. For example, the composition may have a viable probiotic microorganism count of up to about 3 x1013 CFU per gram of food composition, up to about 3x1012 CFU per gram of food composition, or up to about 3x1011 CFU per gram of food composition, or up to about 3x1010 CFU per gram of food composition. CFU is determined using the method provided as part of the European Pharmacopoeial Methods, 2003, Section 2.6.12.

The medicament may comprise at least about 0.001%, alternatively at least about 0.01%, alternatively at least about 0.1%, alternatively at least about 0.5%, and alternatively at least about 1% of the probiotic, by weight of the medicament. As further examples, the medicament may comprise about 99% or less, alternatively about 75% or less, alternatively about 50% or less, alternatively about 25% or less, alternatively about 10% or less, and alternatively about 5% or less of the probiotic, by weight of the medicament.

The compositions used herein may optionally comprise one or more further components. Other components are beneficial for inclusion in the compositions used herein, but are optional for purposes of the invention. Optional components can include crude protein, fat, carbohydrate, vitamins, minerals or a mixture thereof.

In one embodiment, the compositions may comprise, on a dry matter basis, from about 13% to about 50% crude protein, alternatively from about 12% to about 46% crude protein by weight of the composition. The crude protein material may comprise vegetable-based proteins such as soybean, cereals (corn, wheat, etc), cottonseed, and peanut, or animal-based proteins such as casein, albumin, and meat protein. Non-limiting examples of meat protein useful herein include a protein source selected from the group consisting of beef, pork, lamb, poultry, fish, and mixtures thereof.

Furthermore, the compositions may comprise, on a dry matter basis, from about 5% to about 40% fat, alternatively from about 4.5% to about 37% fat, by weight of the composition. Embodiments related to compositions of the invention may further comprise one or more sources of carbohydrate. In one embodiment, the compositions may comprise from about 25%, up to about 60%, by weight of the composition, carbohydrate. Grains or cereals such as rice, corn, milo, sorghum, barley, wheat, and the like are illustrative of ingredients that contribute a substantial amount of carbohydrate. Fiber ingredients also contribute to the total carbohydrate in the diet.

Other ingredients can be included and can comprise active ingredients, such as sources of fiber ingredients, mineral ingredients, vitamin ingredients, polyphenols ingredients, amino acid ingredients, carotenoid ingredients, antioxidant ingredients, fatty acid ingredients, calorie restriction mimetic ingredients, prebiotic ingredients, and still other ingredients. Sources of fiber ingredients can include fructooligosaccharides (FOS), beet pulp, mannanoligosaccharides (MOS), oat fiber, citrus pulp, carboxymethylcellulose (CMC), guar gum, gum arabic, apple pomace, citrus fiber, fiber extracts, fiber derivatives, dried beet fiber (sugar removed), cellulose, a-cellulose, galactooligosaccharides, xylooligosaccharides, and oligo derivatives from starch, inulin, psyllium, pectins, citrus pectin, guar gum, xanthan gum, alginates, gum arabic, gum talha, beta-glucans, chitins, lignin, celluloses, non-starch polysaccharides, carrageenan, reduced starch, soy oligosaccharides, trehalose, raffinose, stachyose, lactulose, polydextrose, oligodextran, gentioligosaccharide, pectic oligosaccharide, and/or hemicellulose.

Sources of mineral ingredients can include sodium selenite, monosodium phosphate, calcium carbonate, potassium chloride, ferrous sulfate, zinc oxide, manganese sulfate, copper sulfate, manganous oxide, potassium iodide, and/or cobalt carbonate. Sources of vitamin ingredients can include choline chloride, vitamin E supplement, ascorbic acid, vitamin A acetate, calcium paastothenate, pantothenic acid, biotin, thiamine mononitrate (source of vitamin B1), vitamin B12 supplement, niacin, riboflavin supplement (source of vitamin B2), inositol, pyridoxine hydrochloride (source of vitamin B6), vitamin D3 supplement, folic acid, vitamin C, and/or ascorbic acid. Sources of polyphenols ingredients can include tea extract, rosemary extract, rosemarinic acid, coffee extract, caffeic acid, turmeric extract, blueberry extract, grape extract, grapeseed extract, and/or soy extract. Sources of amino acid ingredients can include 1 - Tryptophan, Taurine, Histidine, Carnosine, Alanine, Cysteine, Arginine, Methionine, Tryptophan, Lysine, Asparagine, Aspartic acid, Phenylalanine, Valine, Threonine, Isoleucine, Histidine, Leucine, Glycine, Glutamine, Taurine, Tyrosine, Homocysteine, Ornithine, Citruline, Glutamic acid, Proline, and/or Serine. Sources of carotenoid ingredients can include lutein, astaxanthin, zeaxanthin, bixin, lycopene, and/or beta-carotene. Sources of antioxidant ingredients can include tocopherols (vitamin E), vitamin C, vitamin A, plant-derived materials, carotenoids (described above), selenium, and/or CoQ10 (Co-enzyme Q10).

Non-limiting examples of fats useful herein include any ingredient source selected from the group consisting of beef, pork, lamb, poultry, fish, and mixtures thereof; vegetable ingredients, yeast, and/or algae to deliver specific fatty acids including but not limited to arachidonic acid, alpha-linoleic acid, gamma linolenic acid, linoleic acid, eicosapentanoic acid (EPA), docosahexanoic acid (DHA). Sources of calorie restriction mimetic ingredients can include glucose anti-metabolites including 2-deoxy-D-glucose, 5-thio-D-glucose, 3-0 methylglucose, anhydrosugars including 1,5-anhydro-D-glucitol, 2,5-anhydro-D-glucitol, and 2,5-anhydro-D-mannitol, mannoheptulose, avocado extract comprising mannoheptulose, and/or avocado flesh comprising mannoheptulose. Still other ingredients can include beef broth, brewers dried yeast, egg, egg product, flax meal, DL methionine, amino acids, leucine, lysine, arginine, cysteine, cystine, aspartic acid, polyphosphates such as sodium hexametaphosphate (SHMP), sodium pyrophosphate, sodium tripolyphosphate; zinc chloride, copper gluconate, stannous chloride, stannous fluoride, sodium fluoride, triclosan, glucosamine hydrochloride, chondroitin sulfate, green lipped mussel, blue lipped mussel, methyl sulfonyl methane (MSM), boron, boric acid, phytoestrogens, phytoandrogens, genistein, diadzein, L-carnitine, chromium picolinate, chromium tripicolinate, chromium nicotinate, acid/base modifiers, potassium citrate, potassium chloride, calcium carbonate, calcium chloride, sodium bisulfate; eucalyptus, lavender, peppermint, plasticizers, colorants, flavorants, sweeteners, buffering agents, slip aids, carriers, pH adjusting agents, natural ingredients, stabilizers, biological additives such as enzymes (including proteases and lipases), chemical additives, coolants, chelants, denaturants, drug astringents, emulsifiers, external analgesics, fragrance compounds, humectants, opacifying agents (such as zinc oxide and titanium dioxide), anti-foaming agents (such as silicone), preservatives (such as butylated hydroxytoluene (BHT) and butylated hydroxyanisole (BHA), propyl gallate, benzalkonium chloride, EDTA, benzyl alcohol, potassium sorbate, parabens and mixtures thereof), reducing agents, solvents, hydrotropes, solublizing agents, suspending agents (non-surfactant), solvents, viscosity increasing agents (aqueous and non-aqueous), sequestrants, and/or keratolytics.

Referenced herein are trade names for components including various ingredients utilized in embodiments of the invention. The inventors herein do not intend to be limited by materials under a certain trade name. Equivalent materials (e.g., those obtained from a different source under a different name or reference number) to those referenced by trade name may be substituted and utilized in the descriptions herein.

The food compositions can be made using any suitable method known in the art. The probiotic may be added at any suitable point during the process, for example addition to an ingredient mix prior to mixing and forming of the pet food form. Alternatively, the probiotic may be sprayed onto, or dusted onto the final food composition.

### EXAMPLES

The effect of the use of a probiotic on the incidents of vomiting in domestic cats suffering from chronic idiopathic emesis was tested. Cats of at least 2 years of age were included based on the following inclusion criteria as confirmed by the owner;
- Owner reported that they are very bothered/ somewhat bothered by vomiting;
- Owner reported that the primary content of the vomit is material (food undigested or digested, liquid) NOT hairballs;
- Owner reported that the cat vomits ≥ 1/wk;
- Owner reported that the cat is > 2 years of age;
- Owner reported that the cat is in good health;
- Owner reported that the cat is housed primarily indoors (i.e., at least 90% of time is spent inside);
- Owner reported that the food/water bowls is located inside the house;
- Owner reported that the litter box is located inside of the house.
The cats were then screened and excluded on the following criteria;
- Acute or chronic illness and/or taking medication for an acute or chronic illness (e.g.,internal parasites/worms, allergies, cancer, feline immunodeficiency virus, feline leukemia, diabetes, kidney problems/disease including renal failure, pancreas problems/disease including pancreatitis, inflammatory bowel disease, thyroid problems disease including hyperthyroidism, liver problems/disease);
- Any condition that in the opinion of the veterinarian would cause vomiting (e.g., foreign body obstruction, dietary indiscretion, toxicity);
- Fractious behavior.

The selected cats that met the inclusion criteria and were not excluded on the basis of the exclusion criteria (i.e. those suffering from chronic idiopathic emesis) were fed a control (normalized) diet for 5 weeks. The first week was used as a diet transition period to ensure that all cats were on the same diet. The following 4 weeks were used to collect baseline vomiting frequency for the cats on a normalized diet.

Following the 5 week period, cats were randomly assigned to one of three groups. Each group was fed a different diet. The first diet contained no probiotic and acted as a placebo. The second diet comprised *B. animalis* AHC7 at a concentration such that the cat received 1×108 CFU/day. The third diet comprised *B. animalis* AHC7 at a concentration such that the cat received 1×109 CFU/day.

The cats were fed the diet for 12 weeks and frequency of vomiting recorded. The results can be seen in Table 1;

**Table 1**

| Diet Type | % change from baseline vomiting frequency |
|---|---|
| Control | 0 |
| 1 × 10⁸ CFU/cat/day 10 | 10 |
| 1 × 10⁹ CFU/cat/day 20 | 20 |

As can be seen from Table 1, the use of a probiotic reduced the incidence of vomiting in cats suffering from chronic idiopathic emesis. However, in the absence of a probiotic, there was no change in vomiting incidence versus the baseline.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. Probiotic for use in the treatment of chronic idiopathic emesis in a canine or a feline, wherein the probiotic comprises *Bifidobacterium spp,* wherein the bifidobacterium is *Bifidobacterium animalis* AHC 7.

2. Probiotic for use according to claim 1, for the treatment of chronic idiopathic emesis in a feline.

3. Probiotic for use according to claim 2, wherein the feline is a domestic cat.

4. Probiotic for use according to any of the preceding claims, wherein the probiotic is administered to a canine or feline at a concentration of between 1x10⁶ and 1x10¹² CFU/feline or canine/day, preferably between 1x10⁸ and 1x10¹⁰ CFU/feline or canine/day.

5. Probiotic for use according to claim 4, wherein the probiotic is administered at least once per day, preferably at least three times per day.

6. Probiotic for use according to any of the preceding claims, wherein the probiotic is comprised in a food composition intended for ingestion by the feline or canine.

7. Probiotic for use according to claim 6, wherein the food composition is the form of a dry food composition.

8. Probiotic for use according to claim 6, wherein the food composition is in the form of a supplement, preferably selected from a gravy, aqueous composition, yogurt, powder, suspension, chew, treat, or a mixture thereof.

9. Probiotic for use according to claim 6, wherein the food composition comprises crude protein, fat, carbohydrate, vitamins, minerals or a mixture thereof.

10. Probiotic for use according to any of the preceding claims, wherein the probiotic is in the form of a medicament.

## Patentansprüche

1. Probiotikum für die Verwendung bei der Behandlung von chronischer idiopathischer Emesis bei einem Hund bzw. Hundeartigen oder einer Katze bzw. einem Katzenartigen, wobei das Probiotikum *Bifidobacterium spp.* umfasst, wobei es sich bei dem Bifidobacterium um *Bifidobacterium animalis* AHC 7 handelt.

2. Probiotikum für die Verwendung nach Anspruch 1 zur Behandlung von chronischer idiopathischer Emesis bei einer Katze.

3. Probiotikum für die Verwendung nach Anspruch 2, wobei es sich bei der Katze um eine Hauskatze handelt.

4. Probiotikum für die Verwendung nach einem der vorhergehenden Ansprüche, wobei das Probiotikum an einen Hund oder eine Katze bei einer Konzentration zwischen 1x10⁶ und 1x10¹² CFU/Katze oder Hund/Tag, vorzugsweise zwischen 1x10⁸ und 10x10¹⁰ CFU/Katze oder Hund/Tag verabreicht wird.

5. Probiotikum für die Verwendung nach Anspruch 4, wobei das Probiotikum mindestens einmal pro Tag, vorzugsweise mindestens dreimal pro Tag verabreicht wird.

6. Probiotikum für die Verwendung nach einem der vorhergehenden Ansprüche, wobei das Probiotikum in einer Futtermittelzusammensetzung enthalten ist, die zum Verzehr durch die Katze oder den Hund bestimmt ist.

7. Probiotikum für die Verwendung nach Anspruch 6, wobei die Futtermittelzusammensetzung in Form einer Trockenfutterzusammensetzung vorliegt.

8. Probiotikum für die Verwendung nach Anspruch 6, wobei die Futtermittelzusammensetzung in Form eines Supplements vorliegt, vorzugsweise ausgewählt aus einer Soße, einer wässrigen Zusammensetzung, einem Joghurt, einem Pulver, einer Suspension, einer Kau-Stückform, einem Naschwerk-Bissen oder einer Mischung davon.

9. Probiotikum für die Verwendung nach Anspruch 6, wobei die Futtermittelzusammensetzung rohes Protein, Fett, Kohlenhydrat, Vitamine, Mineralien oder eine Mischung davon umfasst.

10. Probiotikum für die Verwendung nach einem der vorhergehenden Ansprüche, wobei das Probiotikum in Form eines Medikaments vorliegt.

## Revendications

1. Probiotique pour son utilisation dans le traitement du vomissement chronique idiopathique chez un canin ou un félin, où le probiotique comprend *Bifidobacterium* spp, où la bifidobactérie *est* Bifidobacterium animalis AHC 7.

2. Probiotique pour son utilisation selon la revendication 1, destiné au traitement du vomissement chronique idiopathique chez un félin.

3. Probiotique pour son utilisation selon la revendication 2, où le félin est un chat domestique.

4. Probiotique pour son utilisation selon l'une quelconque des revendications précédentes, où le probiotique est administré à un canin ou un félin à une concentration comprise entre 1 x 10⁶ et 1 x 10¹² UFC/félin ou canin/jour, préférentiellement entre 1 x 10⁸ et 1 x 10¹⁰ UFC/félin ou canin/jour.

5. Probiotique pour son utilisation selon la revendication 4, où le probiotique est administré au moins une fois par jour, préférentiellement au moins trois fois par jour.

6. Probiotique pour son utilisation selon l'une quelconque des revendications précédentes, où le probiotique est inclus dans une composition alimentaire destinée à l'ingestion par le félin ou le canin.

7. Probiotique pour son utilisation selon la revendication 6, où la composition alimentaire se présente sous la forme d'une composition alimentaire sèche.

8. Probiotique pour son utilisation selon la revendication 6, où la composition alimentaire se présente sous la forme d'un complément, préférentiellement choisi parmi une sauce, une composition aqueuse, un yaourt, une poudre, une suspension, un accessoire à mâcher, une friandise ou l'un de leurs mélanges.

9. Probiotique pour son utilisation selon la revendication 6, où la composition alimentaire comprend une protéine brute, une matière grasse, un hydrate de carbone, des vitamines, des minéraux ou l'un de leurs mélanges.

10. Probiotique pour son utilisation selon l'une quelconque des revendications précédentes, où le probiotique se présente sous la forme d'un médicament.
